# EUROPEAN PATENT APPLICATION

(11) **EP 0 561 744 A1**
(43) Date of publication of application: **22.09.1993**
(21) Application number: 93830048.0
(22) Date of filing: 11.02.1993
(51) Int. Cl.: A61K 31/455, A61K 31/375, A61K 35/72, A61K 31/355

(54) **Preparations containing chromium and vitamins E and C for controlling carbohydrate and lipid metabolism**

(30) Priority: 20.03.1992 IT RM920206
(71) Applicant: SIRC S.p.A. NATURAL & DIETETIC FOODS, I-20090 Caleppio Di Settala (MI) (IT)
(72) Inventor: Littera, Renato, I-10143 Torino (IT)
(74) Representative: Sarpi, Maurizio

(57) **Abstract**

The use of the combination of organic chromium and two vitamins having antioxidant activity such as vitamin E and vitamin C is disclosed for preventing cardiovascular diseases through the regulation of the carbohydrate metabolism and lipid peroxidation.

## Description

The present invention relates to an organic chromium product against cholesterol.
More particularly the invention relates the use of preparations based upon the combination of organic chromium and two vitamins having antioxidant activity such as vitamin E and vitamin C as dietary products forpreventing cardiovascular diseases.

The combination chromium/vitamin C and vitamin E, the use of which is herein disclosed, has been formulated on the base of the effects of the three components to the carbohydrate metabolism and lipid peroxidation which aims in particular at decreasing the cholesterol and triglyceride levels in case they are too high. This product can be supplied in the common dose units such as capsules, tablets and pills by oral use. By way of example a capsule dose will be illustrated where chromium is present in the form of chromium yeast and vitamin E in the form of tocopherol acetate with the addition of compounds known to those skilled in the art. Such products find application as food integrator of diabetic subjects having impaired carbohydrate metabolism, in case of dislipemia and whenever chromium is more requested as in the maternity, lactation and during intense exertions and strains.

### CHROMIUM

There are many scientific evidences showing the essentiality of chromium in the human nourishment, in particular for controlling the carbohydrate and lipid metabolism (J. Lederer: Le Chrome. Cah.Nutr.Diet., XXIV 2. 1989, 112-118) and whenever the diet gives an insufficient contribution, thus requiring an oral supplementation of chromium.
However, in order to carry out its physiological activity the inorganic trivalent chromium has to be converted into an organic low-molecular-weight complex formed of trivalent chromium, nicotinic acid, and aminoacids, socalled GFT, glucose tolerance factor, which has been isolated from brewers' yeast (U. Tischler: (Chrom - ein essentielles Spurenelement. Teil II: Metabolische Funktionen. Vitaminspur 3.2 (1988) 75-82; Hippokrates Verlag, Stuttgart).
Such complex is a by-maker of insulin adapted to strengthen the capability of the latter to be received because of the catalyzed building up of sulphide bridges between the hormone and the sulphydrile radicals of the receptor with the consequence of an improved carbohydrate tolerance and a reduction of the glycemia.

The effects of chromium to carbohydrate metabolism are widely illustrated in the literature.
In experimental animals receiving foods depleted of chromium it has been observed that the supply of chromium caused an increase of the insulin uptake and the development of glucose oxidation processes (U. Tischler: Chrom - ein essentielles Spurenelement. Teil II). In diabetic patients receiving total parenteral nutrition it has been observed that the supply of chromium was able to mitigate the symptoms of the disease and to decrease the hyperglycemia. In another study over victims of accidents receiving total parenteral nutrition, Anderson discloses the case of a patient having a high hyperglycemia even he was daily given 12 µg chromium, and after a further supplementation of 12 µg chromium his glycemia decreased from 450 to 150 mg/dil; when the supplementation of chromium was stopped glycemia returned to high values and decreased after a new supplementation again.
In young patients affected with Turner's syndrome (among which the incidence of diabetes is high), who were daily given brewers' yeast containing 50 µg chromium for 8 weeks, an improvement in glucose tolerance was noted (G. Saner et al.: Alterations of chromium metabolism and effect of chromium supplementation in Turner's syndrome patients. The American Journal of Clinical Nutrition 38: October 1983, pages. 574-578).
In a double-bind crossover study conducted over three groups of subjects with different glucose tolerance (Serum chromium of human subjects: effects of chromium supplementation and glucose. The American Journal of Clinical Nutrition. 41: March 1985, pages 571-577) Anderson et al report a clear improvement in glucose tolerance after daily giving 200 µg chromium in the form of chromium cloride. In addition the authors report an increase in the glycemia level in hypoglycemic subjects treated with the same chromium supplementation.

As far as the effect of chromium to lipid metabolism is concerned, there are several studies over test animals and human subjects indicating a connection between secondary chromium depletion and impairment of the lipid metabolism.
Thus, rats fed with a diet full of carbohydrates and cholesterol and depleted of chromium had a hyperdislipemia, which was considerably decreased by a supplementation of chromium.
In particular the cholesterolemia and the levels of triglicerides and LDL cholesterol were decreased while the levels of HDL cholesterol were increased. Abrahem et al. noticed a lower incidence and consistency of atheroma plaques in hypercholesterolemic rabbits as a consequence of chromium supplementation.
In human experiments it was noted that the oral supplementation of 20 µg chromium to 12 voluntary subjects for three months caused a decrease in the trigliceride levels and an increase in the plasma levels of HDL cholesterol.
The study of Mossop regarding diabetic subjects who were supplemented chromium diet indicated a decrease in the total cholesterolemia and an increase by about 25% in the HDL cholesterol as well as a hypoglycemia.
Further studies discloses the beneficial effects to lipid concentration of the supplementation of brewers' yeast containing the biologically active form of chromium. A significant mutual relation between the appearance of the coronary disease and reduced plasma levels of chromium has been observed in subjects with cardiovascular diseases.
A very little concentration of chromium has been noticed in the aorta, myocardium, kidney and hair of subjects died by infarct with respect to the concentration of chromium in patients died bt other reasons.
On the base of such experimental and clinical evidences it may be possible to come to the conclusion that chromium exerts a protective influence to the appearance and the extension of arteriosclerotic processes through the regulation of the lipid metabolism, thus reducing the death rate by cardiovascular diseases.

Because of the lack of reliable methods for diagnosing the nutrition state regarding chromium it is difficult to determine the right man's need of chromium. However, a number of symptoms indicating a secondary deficiency of chromium such as reduced glucose tolerance, glycosuria, hyperinsulinemia, hypoglycemia, and high levels of triglycerides and cholesterol with decrease of HDL cholesterol have been recognized . A safety range of 50-200 µg/day has been ascertained on the base of the absence of the above mentioned deficiency symptoms in the most of people consuming 50 µg/day chromium with a diet as well as on the use of an amount of about 200 µg/day in long-range tests with human subjects receiving a supplementation of 150 µg/day beside the amount consumed with the diet.
Several studies indicate the possibility of chromium intake below the suggested safe levels mentioned above. Thus, a study of Anderson et Kozlovsky over 216 healthy subjects indicates that the average daily intake of chromium was 33 µg for males and 25 µg for females. More recent data indicates that dietary intake of chromium is lower than 100 µg/day in many diets.

It is then possible that the dietary intake of chromium is not sufficient all the more that the absorption of chromium is very low (0.5-1%) so that several dietary and physiological factors may cause a reduction of the biological levels needed by the organism. On the other hand the appearance of beneficial effects due to a dietary supplementation of chromium over the glucose tolerance and lipemia in several subjects having impaired carbohydrate or lipid metabolism would seem to confirm such possibility.

The need of chromium by the organism can be increased by a number of conditions.
In some dietary and physiological conditions which are generally connected to an impaired glucose metabolism a greater chromium excretion due to a greater mobilization of the same and a reduced renal absorption may occur. In such cases there is a higher request to compensate the greater losses.
Conditions such as nourishment with a high amount of sugar, intense exertions ans strains, maternity and lactation require the greatest amount of chromium for the organism as illustrated below:
- Diets with high amount of sugar: in a study of Kozlowsky et al. over 37 subjects consuming well-balanced diets during 12 weeks and then diets with high levels of pure sugars (from 15% to 35% sugars) a considerable increase of urinary chromium losses was noted, which could bring to a secondary chromium deficiency.
- Heavy strains: in a study conducted over 9 racers who had run for 10 km a high urinary chromium loss was found as indicated by urinary concentrations which were 5 times higher than those before the race.
   It was also found that the urinary chromium excretion in trained subjects was about half the excretion of non-trained subjects, which can be attributed to a partial depletion of chromium deposits in the organism as a consequence of increased chromium losses associated to heavy strains.
- Pregnancy and lactation: it was found during pregnancy that there are urinary chromium losses by 50% higher than those of women being the same age under normal physiological conditions. In addition, a total daily output of chromium in the milk of 0.2 µg was given during lactation.
   Then it seems that maternity is capable to produce a depletion of chromium levels in the organism which can take 3-4 years to return to standard values.

On the other hand there are many scientific evidences proving the essentiality of vitamin E and vitamin C in the human nourishment, in particular due to their antioxidant activity. Both epidemiological and experimental researches indicate that a deficiency of vitamins E and C can be related to the cardiovascular complications of the arteriosclerosis and be an additional factor to the known risk factors (smoke, cholesterol, obesity, a.s.o.)
Researches carried out both over healthy subjects and carriers of different clinical pictures proved a positive relation between plasma concentration of vitamin E and cholesterol.

Researches over healthy subjects and patients having low density lipoproteins and treated with vitamin E allowed to find that vitamin E is divided among the single lipoprotein fractions in proportion to their total lipid content.

Vitamin C plays an important role in many hydroxylation processes and recently its action for transforming cholesterol into biliary acids has been studied by many researchers.
It has been proved that the supplementation of vitamin C increases the hydroxylation of the cholesterol in the carbon in position 7, thus forming 7-alpha-hydroxychotesterol.
It has been proved that vitamins E and C have a favourable action to the reduction of the oxidation of the organism (the socalled oxidative hypothesis of the arteriosclerosis) due to their prevailing antioxidant activity.

The Applicant has experimentally detected that the best results are achieved by daily giving 100-200 µg Cr, 30-60 mg vitamin C and 5-20 mg vitamin E.
A dose unit in the form of a capsule has ben proposed where the content of chromium and the two vitamins by weight is sufficient for an average daily supplementation so that a dosage of a capsule per day is advisable. The following formulation illustrates the present invention, however, without limiting the same.

| ORAL SUPPLEMENTATION CAPSULE | | |
|---|---|---|
| Ingredients | Content of each capsule by 350 mg | Content of each capsule by 100 mg |
| Lactose | 201.5 mg | 57.5 g |
| Chromium yeast | 75 mg | 21.4 g |
| Vitamin C | 50 mg | 14.25 g |
| Tocopherol acetate | 20 mg | 5.7 g |
| Precipitated silica | 3.5 mg | 1.0 g |

### IGREDIENTS OF THE CAPSULE:

Gelatin (USPXX), dye: titanium dioxide (E 171), yellow iron oxide (E 172).

## Claims

1. Use of organic chromium associated with antioxidant vitamins in preparations for preventing cardiovascular diseases.

2. Use of organic chromium associated with antioxidant vitamins in preparations for controlling carbohydrate and lipid metabolism.

3. Use or organic chromium associated with antioxidant vitamins in dietary products for the nourishment of diabetic subjects having impaired carbohydrate metabolism, in case of dislipemia and whenever chromium is more requested.

4. Use of organic chromium associated with antioxidant vitamins as claimed in the preceding claims, characterized in that said antioxidant vitamins are vitamin C and vitamin E.

5. A dietary product for the regulation of the carbohydrate metabolism and the lipid peroxidation comprising organic chromium associated with vitamin E and vitamin C.

6. The dietary product of claim 6, characterized in that organic chromium is present as chromium yeast, and vitamin E as tocopherol acetate.

7. The dietary product of claims 6 and 7, characterized in that the three active components are contained in the dose unit in the following percentages by weight:
- organic chromium: 40-65%
- vitamin C 25-40%
- tocopherol acetate: 8-18%

8. The dietary product of claims 6 to 8, characterized in that the dose unit is a capsule of gelatin comprising:
- lactose 57.5 %
- organic chromium 21.3 %
- vitamin C 14.25%
- tocopherol acetate 5.7 %
- precipitated silica 1.0 %
and admissible dyes.
